# EUROPEAN PATENT SPECIFICATION

(11) **EP 0 697 411 B1**
(45) Date of publication and mention of the grant of the patent: **01.07.1998**
(21) Application number: 95107137.2
(22) Date of filing: 11.05.1995
(51) Int. Cl.: C07D 307/52, A61K 31/34

(54) **Process for the manufacture of pharmaceutical grade ranitidine base**
Verfahren zur Herstellung von Ranitdinbase in pharmazeutischer Reinheit
Procédé pour la préparation de ranitidine de qualité pharmaceutique

(30) Priority: 24.06.1994 US 265308
(43) Date of publication of application: 21.02.1996
(73) Proprietor: Ranbaxy Laboratories Limited, New Delhi 110020 (IN)
(72) Inventor: Khanna, Jag Mohan, 74 Asian Games Village, India 110049 (IN); Kumar, Naresh, 121, South Park, India 110019 (IN); Khera, Brij, G-68, Bali Nagar, India 110015 (IN); Ray, Purna Chandra, India 110070 (IN)
(74) Representative: Cohausz & Florack

(56) References cited:
- EP-A- 0 285 681
- GB-A- 1 565 966
- US-A- 4 128 658
- European Pharmacopoeia

## Description

### FIELD OF THE INVENTION

This invention relates to a manufacturing process for the H₂-antagonist 'ranitidine', that is, N-[2-[[[5-(Dimethylamino)methyl]-2-furanyl]methyl]thio]ethyl-N'-methyl-2-nitro-1,1-ethenediamine in crystalline form having >99.5% purity.

### BACKGROUND OF THE INVENTION

Ranitidine hydrochloride, as described and claimed in British Patent Specification No. 1,565,966 (Apr. 1980) shows potent histamine H₂-blocking activity. A process for preparing ranitidine is known and described in U.S. Patent No 4,128,658 (Dec. 1978) and in British Patent Specification No. 1,565,966 (Apr. 1980). Though a method to prepare crude ranitidine base is described in U.S. Patent No. 4,128,658 (Dec. 1978), see Example 15 thereby, the ranitidine base produced by this method has not been evaluated for clinical application. In experiments following Example 15, the ranitidine base produced thereby is brownish yellow in color and has a purity of less than 97% (HPLC), with related substances being ca. 2%.

### SUMMARY OF THE INVENTION

The present invention provides a process for producing ranitidine base which is crystalline, is of pharmaceutical grade (>99.5% purity), and is convenient to produce on a commercial scale.

According to the invention, a process for preparing pharmaceutical grade ranitidine base comprises reacting a mixture of N-methyl-1-(methylthio)-2-nitroetheneamine and 2-[[[5-(Dimethylamino)methyl-2-furanyl]methyl]thio]ethanamine in water at 40°C to reflux temperature, removing the unreacted starting material and other impurities at a suitable pH (4-5), preferably by washing, separating the ranitidine base at a suitable pH (9-10), removing water, if any, and precipitating ranitidine base from a "suitable organic solvent", and collecting the resulting product. The product so obtained can be crystallized from a "suitable organic solvent" to produce almost white powder of ranitidine base, having a purity of about 99.6% (HPLC), with related substances being less than 0.4%.

### BRIEF DESCRIPTION OF THE DRAWINGS

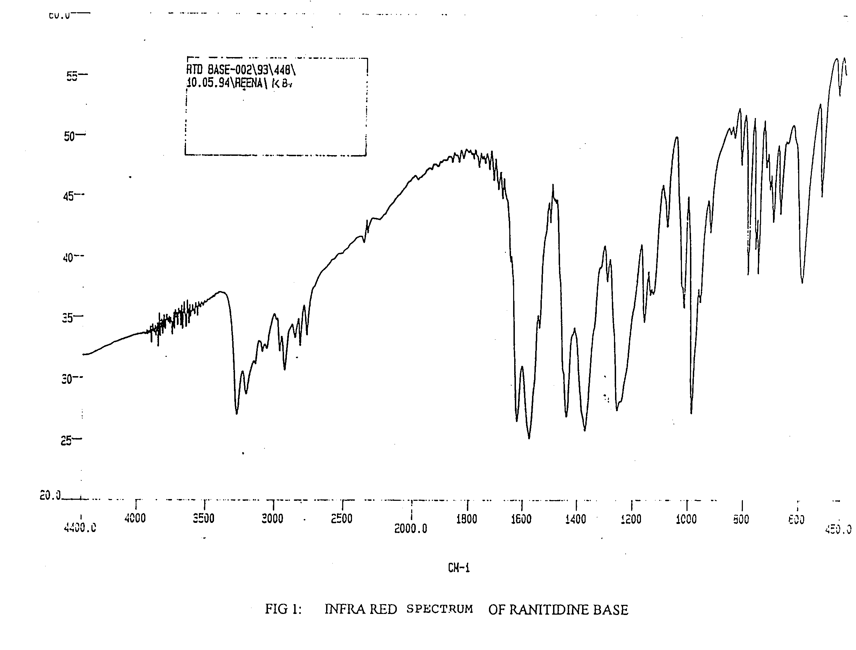

Fig. 1 shows the infra-red spectrum in KBr disc of ranitidine base prepared by the method described in Example 2 below. Ranitidine base prepared this way was also characterized by its proton magnetic resonance spectrum in CDCl₃.

Fig. 2 shows a comparative dissolution profile (dissolution parameters as per USP) of Zantac® (Glaxo) 300mg tablet vs. 300mg tablet of ranitidine base prepared in accordance with the present invention.

### DETAILED DESCRIPTION OF THE INVENTION

In accordance with the present invention, pharmaceutical grade (>99.5% purity) is prepared by reacting a mixture of N-methyl-1-(methylthio)-2-nitroetheneamine and 2-[[[5-(dimethylamino)methyl-2-furanyl]methyl]thio]ethanamine in water at a temperature from about 40°C to reflux temperature, acidifying the solution to remove unreacted starting materials and other impurities, basifying the solution to separate ranitidine base from the solution, removing water if any, and precipitating the ranitidine base from a suitable organic solvent such as a lower alkanol, and collecting the precipitated ranitidine base. Desirably, the precipitated base is dissolved in a suitable organic solvent and crystallized therefrom.

It has been found that if the ranitidine base is prepared under the above defined working conditions, the following advantages are obtained:
a) the product is easily filtrable and can be readily dried;
b) the solvents used are readily recoverable;
c) the product can be crystallized from the same solvent system;
d) the process is economical and convenient to operate on a commercial scale;
e) the process provides a product which has a high degree of purity (>99.5%); and
f) the product is stable.

Generally, the reaction is carried out in water as a medium that has been heated by standard means to a temperature of from about 40°C to reflux temperature, preferably to about 45-70°C, most preferably to about 50°C. This temperature is considerably lower than the reaction temperature of the process described in Example 15 of U.S. 4,128,658. The amount of water is at least 1 part by volume per part of the starting material. Higher amounts of water and generally up to 10 parts by volume can be used. Amounts higher than 10 volumes are not useful from an economical point of view because large size apparatus would be necessary.

The reaction will typically be accomplished within about 3 to about 10 hrs, preferably, within 3-4 hours. However, the length of time required will vary depending on such factors as total volume of solution, size of batch and container, temperature of the reaction, and presence or absence of stirring.

The reaction mixture can be acidified to a pH of about 4-5 by means of mineral acids, preferably hydrochloric acid or sulfuric acid, in different concentrations, or organic acids. Unreacted starting material and other impurities can be washed with any water-immiscible solvents like chlorinated hydrocarbons, aromatic hydrocarbons and ketones at different temperatures. In a preferred embodiment of the invention said water immiscible solvent is dichloromethane, chloroform, or 1,2-dichlorethane.

The reaction mixture can be basified to a pH of 9 to 10 with basifying agents such as alkali metal carbonates, bicarbonates and hydroxides, wherein preferred alkali metals are sodium or potassium. Further preferred basifying agents include ammonium carbonate, ammonium bicarbonate, or ammonium hydroxide.

The term "suitable organic solvent" means any lower alkanol and includes those primary, secondary and tertiary alcohols having from one to six carbon atoms, ketones, esters and aliphatic or aromatic hydrocarbons having from one to ten carbon atoms. According to a preferred embodiment of the invention, said suitable organic solvents are n-propanol, isopropanol, isobutanol, n-butanol, ethylmethyl ketone, 4-methylpentan-2-one, ethylacetate, n-butylacetate, toluene, or cyclohexane. Mixtures of two or more solvents are also contemplated.

It is also desirable to filter the solution prior to crystal formation in order to remove any insoluble, particulate material. The solutions can be cooled to -10° to 50°C, preferably to 0 to 30°C, most preferably to about 10°C, before precipitation by standard procedures well known in the art.

Addition of a miscible solvent such as hexane, petroleum ether, toluene, ethyl acetate and n-butyl acetate to the solution can be advantageously used to complete crystallazitation.

Methods known in the art may be used with the process of this invention to enhance any aspect of this process. For example, the solution may be seeded with one or more crystals of ranitidine base prior to the initiation of product crystallization.

Generally, after cooling, the product can be collected by any standard method known in the art such as by filtration, filtration under vacuum, or decantation and drying. Typically, this product will be collected by filtration when any of the solvents within the scope of this process are used.

Ranitidine base prepared as above may further be crystallized using conditions and solvents for crystallizations similar to those described above. Mixtures of two or more solvents are also contemplated. Suitable solvents for further crystallization are alkanols. Preferred additional organic solvents are a primary alcohol, a secondary alcohol, a tertiary alcohol, a ketone, an ester, a saturated hydrocarbon, or an unsaturated hydrocarbon. Similar to the crystallizations above, addition of a further organic solvent such as hexane or petroleum ether to the solutions can be advantageously used to complete crystallization.

Histamine H₂ antagonist activity of ranitidine base prepared as above has been studied in-vitro by using guinea pig atrium and in-vivo on aspirin induced gastric lesions in conscious rats. Both ranitidine base as well as Form 2 ranitidine hydrochloride were found equi-potent in these tests.

Acute toxicity studies were conducted in mice. From the results of the acute toxicity studies it is concluded that ranitidine base prepared as above is as safe as Form 2 ranitidine hydrochloride.

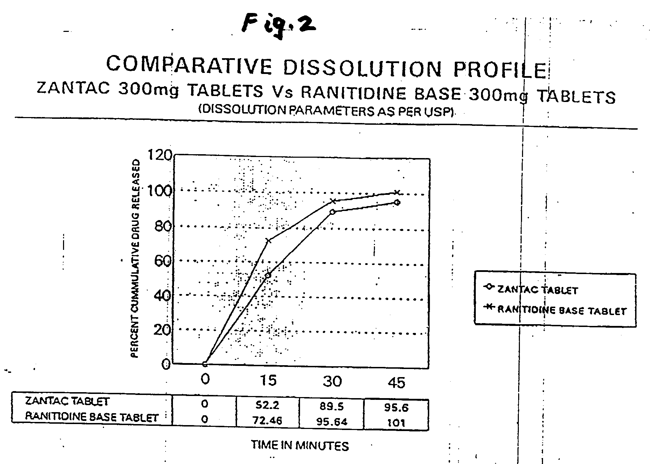

Ranitidine base prepared as above has been used in formulating the following products:
i) Ranitidine base tablets in two strengths of 150 mg and 300 mg, and
ii) Ranitidine base injection containing 25 mg/ml of the drug. Both strengths of the tablet formulation have been found to have comparable dissolution rates in in-vitro performance to that of Zantac^{R} (Glaxo) which contains Form 2 ranitidine hydrochloride. See Fig. 2.

The accelerated stability of these formulations is satisfactory and long term room temperature studies are currently underway.

The following specific examples are presented to illustrate the inventive process.

### EXAMPLE 1

### Preparation of Ranitidine Base

2-[[[5-(Dimethylamino)methyl-2-furanyl]methyl]thio] ethanamine (50 g) and N-methyl-1-(methylthio)-2-nitroetheneamine (40 g) in water (235 ml) was stirred and heated at 45-40°C. The solution was stirred further for 3-4 hrs. It was acidified with hydrochloric acid and extracted with chloroform. The solution was basified with potassium carbonate and ranitidine base was separated. The water, if any, was removed by azeotropic distillation under reduced pressure at 40-45°C using isopropanol. The resultant solution was cooled to 10°C and n-hexane (500ml) was added. Ranitidine base was filtered off and dried under vacuum to give the crude product (66.2g), m.p. 68-70°C; purity = 99.4% (HPLC); IR(KBr): 3280, 3200, 2820, 2774, 1620, 1580, 1440, 1370, 1258, 1130, 1020, 990, 820, 790, 760, 680 and 600 cm⁻¹; δ_{H}(CDCl₃):2.23[6H,s, -N(CH₃)₂], 2.75(2H,t, -SCH₂CH₂), 2.85(3H,br d, CH₃NH-), 3.3 (2H,2t, -CH₂NH-),3.4 (2H,s, >NCH₂), 3.7 (2H,s,CH₂ bridge linking), 6.0-6.2 (2H,AB,furan) and 6.5 (1H,s,=CHNO₂).

### EXAMPLE 2

The process of Example 1 was repeated at a commercial scale, using 2-[[[5-(Dimethylamino)methyl-2-furanyl]methyl]thio]ethanamine (35kg) and N-methyl-1-(methylthio)-2-nitroetheneamine (28kg) to give crude ranitidine base (46.5 kg), m.p. 68-70°C; purity - 99.1% (HPLC).

### EXAMPLE 3

The process of Example 1 was repeated at 25g scale, using a mixture of ethyl acetate and isopropanol instead of isopropanol to give ranitidine base (34.5g), m.p. 67-69°C; purity = 98.8% (HPLC).

### EXAMPLE 4

2-[[[5-(Dimethylamino)methyl-2-furanyl]methyl]thio]ethanamine (25g) and N-methyl-1-(methylthio)-2-nitroetheneamine (18g) in water (35ml) was stirred and heated at 50°C. The solution was stirred further for 3-4 hrs. It was acidified with sulfuric acid and extracted with chloroform. The solution was basified with potassium carbonate and ranitidine base was separated. The water, if any, was removed by azeotropic distillation under reduced pressure at 40-45°C using isopropanol. The resultant solution was cooled to 10°C and n-hexane (250ml) was added. Ranitidine base was filtered off and dried under vacuum to give the crude product (30g), m.p. 67-69°C, purity =99.1% (HPLC)

### EXAMPLE 5

2-[[[5-(Dimethylamino)methyl-2-furanyl]methyl]thiol]ethanamine (25g) and N-methyl-1-(methylthio)-2-nitroetheneamine (20g) in water (118ml) was stirred and heated at 50°C The solution was stirred further for 3-4 hrs. It was acidified with sulfuric acid and extracted with chloroform. The solution was basified with potassium carbonate and ranitidine base was extracted with chloroform and worked up. Isopropanol was added to the residue, cooled to 10°C, and n-hexane (250 ml) was added. Ranitidine base was filtered off and dried under vacuum to give the crude product (33 g), m.p. 67-69°C; purity=99% (HPLC)

### EXAMPLE 6

### Crystallization of ranitidine base

Ranitidine base (25g) prepared earlier was crystallized by dissolving in isopropanol at 35-40°C and precipitating from n-hexane. The slurry was cooled to 10°C and stirred for 1-2 hrs. The product was filtered off, washed with n-hexane, and dried to give pure ranitidine base (21g), m.p. 71-72°C; purity =99.6% (HPLC)

### EXAMPLE 7

The process of Example 5 was repeated using isobutanol instead of isopropanol at 5g scale to give pure ranitidine base (3.9g), m.p. 71-72°C; purity = 99.8% (HPLC)

### EXAMPLE 8

Ranitidine base (150g) prepared earlier was crystallized by dissolving in toluene at 38-40°C. The resultant solution was cooled to 10°C and the slurry was stirred for 1 hour at 10-12°C. The product was filtered off, washed with pre-cooled toluene and dried under vacuum to give pure ranitidine base (135g), m.p. 70-71°C; purity = 99.8% (HPLC)

### EXAMPLE 9

The process of Example 7 was repeated, using ethyl acetate instead of toluene to give pure ranitidine base (137g), m.p. 70-71°C; purity = 99.8% (HPLC)

### EXAMPLE 10

The process of Example 7 was repeated, using 4-methylpentan-2-one instead of toluene to give pure ranitidine base (120g), m.p. 71-72°C; purity = 99.7% (HPLC)

It is believed that the higher purity ranitidine base produced by the process of the present invention in comparison to the method described in Example 15 of U.S. 4,128,658, may be attributed to one or more of the following combination of factors:
(1) reaction under higher dilution of reaction medium, i.e., water, thereby producing fewer impurities;
(2) higher molar concentration (1.157:1) of one of the starting materials, i.e., N-methyl-1-(methylthio)-2-nitroetheneamine, with respect to 2-[[[5-(Dimethylamino)methyl-2-furanyl]methyl]thio]ethanamine, as against a ratio of 1.036:1 in Example 15 of U.S. 4,128,658;
(3) shorter reaction time (ca. 3-4 hours) than in Example 15 of U.S. 4,128,658, which reduces the quantity of impurities formed, and monitoring of the completion of the reaction by TLC;
(4) removal of excess starting materials and impurities by extraction at a suitable pH; and
(5) recrystallization of the crude ranitidine base to give a higher purity final product.

While the invention has been described by reference to specific embodiments, this was for purposes of illustration only. Numerous alternative embodiments will be apparent to those skilled in the art and are considered to be within the scope of the invention.

## Claims

1. A process for preparing ranitidine base of a purity of 99.5% or higher comprising reacting a mixture of N-methyl-1-(methylthio)-2-nitroetheneamine and 2-[[[5-(dimethylamino)methyl-2-furanyl)methyl]thio]ethanamine in a water solution at a temperature from about 40°C to reflux temperature, adding an acid to said solution to remove unreacted starting materials and impurities, adding a basifying agent to said solution to separate ranitidine base from said solution, removing water if any and precipitating ranitidine base from an organic solvent selected from the group consisting of primary, secondary and tertiary alcohols having from one to six carbon atoms, ketones, esters and aliphatic or aromatic hydrocarbons having from one to ten carbon atoms, and collecting said precipitated ranitidine base.

2. The process of claim 1 wherein said addition of an acid to the solution is performed until a pH of 4 to 5 is adjusted in said solution.

3. The process of claim 1 or 2 wherein said acid is a mineral acid or an organic acid.

4. The process of claim 3 wherein said acid is hydrochloric acid or sulfuric acid.

5. The process of claim 4 further comprising washing said unreacted starting material and other impurities with a water-immiscible solvent.

6. The process of claim 5 wherein said water immiscible solvent is a chlorinated hydrocarbon, aromatic hydrocarbon, or ketone.

7. The process of claim 6 wherein said water-immiscible solvent is dichloromethane, chloroform, or 1,2-dichloroethane.

8. The process of claim 1 wherein said addition of a basifying agent to the solution is performed until a pH of 9 to 10 is adjusted in said solution.

9. The process of claim 8 wherein said basifying agent is an alkali metal carbonate, alkali metal bicarbonate, or alkali metal hydroxide.

10. The process of claim 9 wherein said alkali metal is sodium or potassium.

11. The process of claim 8 wherein said basifying agent is ammonium carbonate, ammonium bicarbonate, or ammonium hydroxide.

12. The process of claim 1 wherein said solvent is n-propanol, isopropanol, isobutanol, n-butanol, ethylmethyl ketone, 4-methyl pentan-2-one, ethyl acetate, n-butyl acetate, toluene, or cyclohexane.

13. The process of claim 1 further comprising adding an additional organic solvent to complete crystallization of said ranitidine base product.

14. The process of claim 13 wherein said additional organic solvent is hexane, petroleum ether, toluene, ethyl acetate, or n-butyl acetate.

15. The process of claim 1 further comprising dissolving said collected ranitidine base in an additional organic solvent and crystallizing ranitidine base from said additional organic solvent.

16. The process of claim 15 wherein said additional organic solvent is an alkanol.

17. The process of claim 15 wherein said additional organic solvent is a primary alcohol, a secondary alcohol, a tertiary alcohol, a ketone, an ester, a saturated hydrocarbon, or an unsaturated hydrocarbon.

18. The process of claim 15 further comprising adding a further organic solvent to complete crystallization of said ranitidine base from said additional organic solvent.

19. The process of claim 18 wherein said further organic solvent is hexane or petroleum ether.

## Patentansprüche

1. Verfahren zur Herstellung von Ranitidin-Base mit einer Reinheit von 99,5% oder höher, welches das Umsetzen eines Gemischs aus N-Methyl-1-(methylthio)-2-nitroethenamin und 2-[[[5-(dimethylamino)methyl-2-furanyl]methyl]thio]ethanamin in einer wässrigen Lösung bei einer Temperatur von etwa 40°C bis Rückflußtemperatur, das Zugeben einer Säure zu der Lösung, um nicht umgesetztes Ausgangsmaterial und Verunreinigungen zu entfernen, das Zugeben basisch machenden Mittels zu der Lösung, um die Ranitidin-Base von der Lösung zu trennen, das Entfernen von gegebenenfalls vorhandenem Wasser und das Fällen von Ranitidin-Base aus einem organischen Lösungsmittel, ausgewählt aus der Gruppe, bestehend aus primären, sekundären und tertiären Alkoholen mit ein bis sechs Kohlenstoffatomen, Ketonen, Estern und aliphatischen oder aromatischen Kohlenwasserstoffen mit ein bis zehn Kohlenstoffatomen und Sammeln der ausgefällten Ranitidin-Base umfaßt.

2. Verfahren nach Anspruch 1, worin die Zugabe einer Säure zu der Lösung so lange durchgeführt wird, bis ein pH-Wert von 4 bis 5 in der Lösung eingestellt ist.

3. Verfahren nach Anspruch 1 oder 2, worin die Säure eine Mineralsäure oder eine organische Säure ist.

4. Verfahren nach Anspruch 3, worin die Säure Salzsäure oder Schwefelsäure ist.

5. Verfahren nach Anspruch 4, daß ferner ein Auswaschen des nicht umgesetzten Ausgangsmaterials und weiterer Verunreinigungen mit einem nicht mit Wasser mischbaren Lösungsmittel umfaßt.

6. Verfahren nach Anspruch 5, worin das mit Wasser nicht mischbare Lösungsmittel ein chlorierter Kohlenwasserstoff, aromatischer Kohlenwasserstoff oder Keton ist.

7. Verfahren nach Anspruch 6, worin das mit Wasser nicht mischbare Lösungsmittel Dichlormethan, Chloroform oder 1,2-Dichlorethan ist.

8. Verfahren nach Anspruch 1, worin die Zugabe eines die Lösung basisch machenden Mittels zu der Lösung so lange erfolgt, bis ein pH-Wert von 9 bis 10 in der Lösung eingestellt ist.

9. Verfahren nach Anspruch 8, worin das basisch machende Mittel ein Alkalimetallcarbonat, Alkalimetallbicarbonat oder Alkalimetallhydroxid ist.

10. Verfahren nach Anspruch 9, worin das Alkalimetall Natrium oder Kalium ist.

11. Verfahren nach Anspruch 8, worin das basisch machende Mittel Ammoniumcarbonat, Ammoniumbicarbonat oder Ammoniumhydroxid ist.

12. Verfahren nach Anspruch 1, worin das Lösungsmittel n-Propanol, Isopropanol, Isobutanol, n-Butanol, Ethylmethylketon, 4-Methylpentan-2-on, Ethylacetat, n-Butylacetat, Toluol oder Cyclohexan ist.

13. Verfahren nach Anspruch 1, daß ferner die Zugabe eines weiteren organischen Lösungsmittels zur Vervollständigung der Kristallisation der Ranitidin-Base umfaßt.

14. Verfahren nach Anspruch 13, worin das zusätzliche organische Lösungsmittel Hexan, Petrolether, Toluol, Ethylacetat oder n-Butylacetat ist.

15. Verfahren nach Anspruch 1, daß ferner ein Lösen der gesammelten Ranitidin-Base in einem weiteren organischen Lösungsmittel sowie das Kristallisieren von Ranitidin-Base aus dem weiteren organischen Lösungsmittel umfaßt.

16. Verfahren nach Anspruch 15, worin das zusätzliche organische Lösungsmittel ein Alkanol ist.

17. Verfahren nach Anspruch 15, worin das zusätzliche organische Lösungsmittel ein primärer Alkohol, ein sekundärer Alkohol, ein tertiärer Alkohol, ein Keton, ein Ester, ein gesättigter Kohlenwasserstoff oder ein ungesättigter Kohlenwasserstoff ist.

18. Verfahren nach Anspruch 15, daß ferner die Zugabe eines weiteren organischen Lösungsmittel zur Vervollständigung der Kristallisation der Ranitidin-Base aus dem zusätzlichen organischen Lösungsmittel umfaßt.

19. Verfahren nach Anspruch 18, worin das weitere organische Lösungsmittel Hexan oder Petrolether ist.

## Revendications

1. Procédé de préparation de ranitidine base de pureté de 99.5 % ou plus comprenant les étapes de mise en réaction d'un mélange de N-méthyl-1-(méthylthio)-2 nitroéthéneamine et de 2- [[[5- (diméthylamino)méthyl- 2-furanyl] méthyl]thio]- éthanamine dans une solution aqueuse à une température comprise entre 40°C et la température de reflux, addition d'un acide à ladite solution pour éliminer les impuretés et les produits de départ n'ayant pas réagis, addition d'un agent basique à ladite solution pour séparer la ranitidine base de ladite solution, élimination de l'eau s'il y en a et précipitation de la ranitidine base à partir d'un solvant organique choisi dans le groupe constitué par les alcools primaires, secondaires ou tertiaires ayant de un à six atome de carbone, les cétones, les esters et les hydrocarbures aromatiques ou aliphatiques ayant de un à dix atomes de carbone, et récupération de ladite ranitidine base.

2. Procédé selon la revendication 1 dans lequel ladite addition d'un acide à la solution est effectuée jusqu'à obtention d'un pH de 4 à 5.

3. Procédé selon la revendication 1 ou la revendication 2 dans lequel ledit acide est un acide minéral ou un acide organique.

4. Procédé selon la revendication 3 dans lequel l'acide est l'acide chlorhydrique ou l'acide sulfurique.

5. Procédé selon la revendication 4 comprenant en outre le lavage desdits produits de départ non-réagis et les autres impuretés avec un solvant non miscible à l'eau.

6. Procédé selon la revendication 5 dans lequel ledit solvant non miscible à l'eau est un hydrocarbure chloré, un hydrocarbure aromatique ou une cétone.

7. Procédé selon la revendication 6 dans lequel le solvant non miscible à l'eau est le dichlorométhane, le chloroforme ou le 1,2-dichloroéthane.

8. Procédé selon la revendication 1 dans lequel ladite addition d'un agent basique à la solution est effectuée jusqu'à obtention d'un pH de 9 à 10.

9. Procédé selon la revendication 8 dans lequel l'agent basifiant est un carbonate de métal alcalin, un bicarbonate de métal alcalin ou un hydroxyde de métal alcalin.

10. Procédé selon la revendication 9 dans lequel le métal alcalin est le sodium ou le potassium.

11. Procédé selon la revendication 8 dans lequel l'agent basifiant est le carbonate d'ammonium, le bicarbonate d'ammonium, ou l'hydroxyde d'ammonium.

12. Procédé selon la revendication 1 dans lequel ledit solvant est le n-propanol, l'isopropanol, l'isobutanol, le n-butanol, l'éthylméthyl cétone, le 4-méthyl pentan-2-one, l'acétate d'éthyle, l'acétate de n-butyle, le toluène ou le cyclohexane.

13. Procédé selon la revendication 1 comprenant de plus l'addition d'un solvant organique additionnel pour compléter la cristallisation de ladite ranitidine base.

14. Procédé selon la revendication 13 dans lequel ledit solvant organique additionnel est l'hexane, l'éther de pétrole , le toluène, l'acétate d'éthyle, ou l'acétate de n-butyle.

15. Procédé selon la revendication 1 comprenant en outre une étape de dissolution de ladite ranitidine base récupérée dans un solvant organique additionnel et cristallisation de la ranitidine base à partir dudit solvant organique additionnel.

16. Procédé selon la revendication 15 dans lequel ledit solvant organique additionnel est un alcanol.

17. Procédé selon la revendication 15 dans lequel le dit solvant organique additionnel est un alcool primaire, un alcool secondaire, un alcool tertiaire, une cétone , un ester, un hydrocarbure saturé, ou un hydrocarbure non sature.

18. Procédé selon la revendication 15 comprenant en outre l'étape d'addition d'un autre solvant organique pour mener à bien la cristallisation de ladite ranitidine à partir dudit solvant organique additionnel.

19. Procédé selon la revendication 18 dans lequel l'autre solvant organique est l'hexane ou l'éther de pétrole.
